# EUROPEAN PATENT SPECIFICATION

(11) **EP 2 890 295 B1**
(45) Date of publication and mention of the grant of the patent: **20.09.2023**
(21) Application number: 13762930.9
(22) Date of filing: 27.08.2013
(51) Int. Cl.: A61B 5/107, A61B 5/103, A61F 5/00, A61F 2/00, G06T 1/00

(54) **METHOD AND DEVICE FOR ORDERING A CUSTOM ORTHOPEDIC DEVICE**
VERFAHREN UND VORRICHTUNG ZUM ANFORDERN EINER ANGEPASSTEN ORTHOPÄDISCHEN VORRICHTUNG
MÉTHODE ET DISPOSITIF POUR COMMANDER UN DISPOSITIF ORTHOPÉDIQUE SUR MESURE

(30) Priority: 29.08.2012 US 201261694474 P; 29.08.2012 US 201261694314 P
(43) Date of publication of application: 08.07.2015
(73) Proprietor: Össur HF, 110 Reykjavik (IS)
(72) Inventor: TAYLOR, Jason, Robert, Trabuco Canyon, California 92679 (US); BROOKOVER, Derek, Irvine, California 92620 (US)
(74) Representative: Klunker IP Patentanwälte PartG mbB
(86) International application number: PCT/US2013/056896
(87) International publication number: WO 2014/036029

(56) References cited:
- US-A1- 2009 088 674
- US-A1- 2010 268 138
- US-A1- 2011 166 435

## Description

### FIELD OF THE DISCLOSURE

The present disclosure relates to a method reducing image and limb misalignment in capturing an image of the limb for ordering a custom orthopedic device for a joint of the limb and a device for ordering a custom orthopedic device and a computer program product in the form of an ordering application software for execution of the method on a portable device.

### BACKGROUND

To provide customized support for a j oint, a clinician may provide a patient with a custom fitted orthopedic device adapted to the specific anatomical dimensions of the individual patient. A common orthopedic device for customization is a knee brace. A patient will typically obtain a customized brace through a clinician having the expertise to assure that the orthopedic device fits the patient properly.

A clinician can prepare the brace himself, or order a custom orthopedic device remotely through the mail or by submitting an order over mail, phone, fax or the Internet. During the ordering process, the clinician typically provides the manufacturer or seller ("provider") with an image of a portion of the limb including the joint and measurements of the limb around the joint. The custom orthopedic brace is produced based on the submitted image of the limb and measurements. The provider may require the image of the limb be captured at a certain orientation, angle, height, and distance relative to the limb to ensure that the captured image accurately portrays the dimensions and proportions of the limb. Appropriate tags, reference indicia and reference markings of anatomy are often placed on the limb to identify the patient, the limb, and any other necessary information if the photo is misplaced from an order form.

It is undesirable for the picture to be taken when the camera is at an angle relative to the limb (angle normal to the line of progression of the limb) or when the limb is not aligned with the center of the image since such an image would inaccurately portray the dimensions and proportions of the limb. Producing a custom orthopedic brace based on such a misaligned image results in a poorly fitting brace. Likewise, poor resolution or lack of indicia applied on the limb may impede the producer in fully understanding the contours of the patient's limb.

Using a conventional camera, the photographer must estimate or otherwise measure the specified distance between the camera and the limb, the specified portion of the limb to capture in the image, and the appropriate orientation of the camera relative to the limb. Since the conventional camera does not provide feedback about the angle or orientation at which the camera is held, it is difficult for the photographer to determine whether the image of the limb being captured meets the requirements of the manufacturer or seller without additional aids.

The patient may need to use different devices to complete the entire ordering process. If the image of the limb is captured with a conventional digital camera, the image must then be transferred to a computer before the order and image can be uploaded over the Internet to the server of the manufacturer or seller.

While providing a photo is useful in understanding the patient's anatomy, dimensional measurements are likewise required. Various forms are required for completion by the clinician to determine measurement data and patient personal information. Other forms require the clinician to indicate brace models, features, accessories, colors, etc. From the requirement for forms and a photo, the ordering process both complicated and risks a mismatch of documents for the order.

The features of the disclosure provide a solution to the need to reduce image and limb misalignment and improve the ease of capturing an image of the limb and of ordering a custom orthopedic device without multiple forms.

Various types of calipers are known for measuring different objects. A caliper must be properly applied against the object to take the desired measurement, and when measuring the thickness of between two sides of an object, a caliper must be held at right angles to the piece.

In the field of orthopedics, calipers are used for measuring a knee and a leg for a knee brace. As illustrated in Fig. 7, an exemplary method for measuring a knee includes using a marker to apply landmarks on the patient's leg such as at the medial joint space, and along the tibial crest at the tibial tuberosity and a distance below which is depicted in Figs. 7A and 7B. Referring to Fig. 7C, a conventional caliper 102 comes into use in the method by taking the M-L (medial-lateral) measurement 109 at the joint space against the patient's condyles. The caliper 102 includes jaws 104, 106 snugly adjusted against the patient's skin to obtain the measurement and are left in place as the measurement is recorded along the scale 108.

In using the mid patella as a reference point, as depicted in Fig. 7D, above and below circumference measurements 105, 107 are taken at specified distances, and a caliper may also obtain M-L measurements at these locations as well. Next, with the patient standing in a weight bearing position facing a camera, the height of the camera is adjusted at a mid-patella level, and the camera is positioned so it is approximately a specified distance away from the patient. The image captures the leg a specified distance above and below mid patella and is taken of the anterior side of the knee, and an additional image is taken of the lateral side of the knee, as shown in Figs. 7E and 7F.

The conventional caliper may obtain the M-L measurement and other devices have jaws that slide relative to one another and are indexed to a scale located along a shaft. The clinician must adjust one of the jaws relative to the other jaw, manually move the jaw to press against the patient's soft tissue, and manually removes the jaws from the soft tissue upon completion of the measurement. The force against the soft tissue may exceed comfort levels or may be inconsistently applied from patient to patient.

The conventional caliper 102 may have narrow jaws 104, 106 which may shift over the soft tissue due to their narrow width and may cause discomfort because of sharp tips of the jaws. The jaws are typically elongated and straight, and provide little or any space between areas of the soft tissue within the jaws for adjustment by a clinician. A patient's condyles may have an irregular shape and the narrow jaws of fail to provide an accurate measurement of M/L due to condyle irregularity. In variations, conventional calipers may use contoured "cup-shaped" contact point which extend over the condyles but limit the clinician for placement over the condyles.

Due to a variety of leg shapes, sizes and soft tissue amount, conventional calipers provide inconsistent compression over patients' soft tissue due in part to human error by clinician. The inconsistent compression results in improper measurements for the eventual knee brace and may lead to inferior customization of the knee brace.

US 2010/268138 A1 discloses a custom device such as braces and casts used to protect a portion of a body during recovery. A method for fabricating the custom device includes: marking a body with reference points and/or other indicators; obtaining multiple images of the body from multiple angles; using the images to determine the contours of the body; and locating and using the other markings to design the custom device.

US 2011/166435 A1 discloses a surgical orientation system used to assist a surgeon to orient a prosthetic component relative to a patient's anatomy during surgery. The system includes: an implement for releasable attachment of a prosthetic component; an electronic orientation monitor attached to the implement; and a brace. The brace is releasably attachable to the patient so as to define a reference point relative to the patient's anatomy. The reference point is external of the patient and includes at least one surface defining a reference plane that is used to orient the monitor into a reference orientation to calibrate the monitor. The surgeon then manipulates the implement so that the prosthetic component is in the desired position relative to the patient. The monitor provides an indication to the surgeon when a subsequent orientation of the monitor has a predefined relationship relative to the reference orientation. Upon receiving the indication, the surgeon inserts the prosthetic component into the patient.

US 2009/088674 A1 discloses a method and system for designing a patient-specific orthopedic surgical instrument including coupling a knee sleeve to a leg of the patient, the knee sleeve including sensors configured to generate sensor data indicate of the position of the respective sensor. The design of the patient-specific orthopedic surgical instrument includes determining angulation data indicative of the angulation of the knee based on the sensor data; and generating medical image(s) of the knee; wherein the patient-specific orthopedic surgical instrument is designed based on the angulation data and the medical image(s).

### SUMMARY

It is an object of the present invention to provide a method reducing image and limb misalignment in capturing an image of the limb for ordering a custom orthopedic device for a joint of the limb and device for ordering a custom orthopedic device and computer program product in the form of an ordering application software for execution of the method on a portable device, by which image and limb misalignment in capturing an image of the limb, such as a leg, for ordering a custom orthopedic device for a joint, such as a knee joint, can be reduced to improve the customization of the custom orthopedic device.

The object is achieved by the features of independent claim 1 and 12, respectively. Further embodiments are defined in the respective dependent claims.

According to a variation, the first and second distances are the same and referenced from a knee axis line. The distances above and below the joint may be aligned with the depth of field guideline in the viewfinder image before capturing the image.

The method may also include calibrating the image sensor of the portable device.

The method may include executing an ordering application, determining whether the ordering application has been previously executed. The image sensor may be calibrated upon the determination that the ordering application has not been previously executed. The image of the limb enables capture upon the determination that the ordering application has been previously executed.

The method may include reviewing the captured image of the limb and selecting a custom orthopedic device configuration. The step of reviewing the captured image of the limb includes viewing the captured image with an overlaid depth of field guideline to confirm the captured portion of the limb satisfies the overlaid depth of field guideline. The method may also include entering basic patient information into the portable device including measurements of the limb at various locations on the limb. The captured image may be overlaid with the basic patient information. The overlaid captured image may be stored in the portable device.

The method can involve configuring the custom orthopedic device, reviewing the order, and storing the order in a memory of the portable device. At least one previous order may be stored in the memory of the portable device. The order may be transmitted as an e-mail containing the patient information and the saved, captured image of the limb.

The method may include a login page requiring clinician and patient input. Upon entry of the information on the login page, the user is directed to an order configuration home screen or page. From the home screen, the user may select many pages for making the customized order. The user may first select the image capture and input measurements, followed by entering patient information, orthopedic device (brace) configuration, and any other order information. The user is not limited to a sequence of page use, other than upon entry of all data fields and appropriate image capture, the order is sent to the provider.

The processor can be further configured to calibrate the image sensor by setting the image sensor to a first resolution and a first zoom level. At least one image of anatomical landmarks or markings on the limb is captured. A three-dimensional model of the limb is generated from the markings along with circumferential measurements.

### BRIEF DESCRIPTION OF THE DRAWINGS

The device and method for ordering a custom orthopedic device is described with reference to the accompanying drawings which show preferred embodiments according to the device described herein.
Fig. 1 is an overview of the steps in an embodiment of the custom orthopedic device ordering method.
Fig. 2 shows a portrait orientation of a device for ordering the custom orthopedic device with respect to three axes.
Fig. 3 is an example of the indication provided to the user of the preferred orientation of the device.
Fig. 4 shows the device having a viewfinder image overlaid with a depth of field guideline.
Fig. 5 is an example of the captured image stored with an identification label.
Fig. 6 illustrates is a flowchart of an embodiment of the custom orthopedic device ordering method using a three-dimensional model of the limb.

### DETAILED DESCRIPTION OF VARIOUS EMBODIMENTS

A better understanding of different embodiments of the disclosure may be had from the following description read with the accompanying drawings in which like reference characters refer to like elements.

The embodiments of the method 2 and device 4 disclosed enable the user to easily capture accurate images of the limb and complete the ordering process on a single device. In an embodiment, the method 2 is implemented in an application executed on a portable device. The application guides the user through the ordering and image capturing process. Such a device and process reduces misalignment issues while integrating picture capturing and ordering into a single device.

The device used to capture the image and order the custom orthopedic brace may be any device having a display and an image sensor such as a mobile phone (iPhone^{®}, Android^{®} phone, Blackberry^{®}, Windows^{®} phone, etc.), a tablet (iPad^{®}, Android^{®} tablet, Windows^{®} tablet etc.), a personal digital assistant (PDA), a computer, or any other portable device.

The preferred device 4 has an image sensor, a display, a processor, a gyroscope and/or accelerometer, memory, and a communication interface to allow communication of the order directly from the device over a network to a server of the manufacturer or seller.

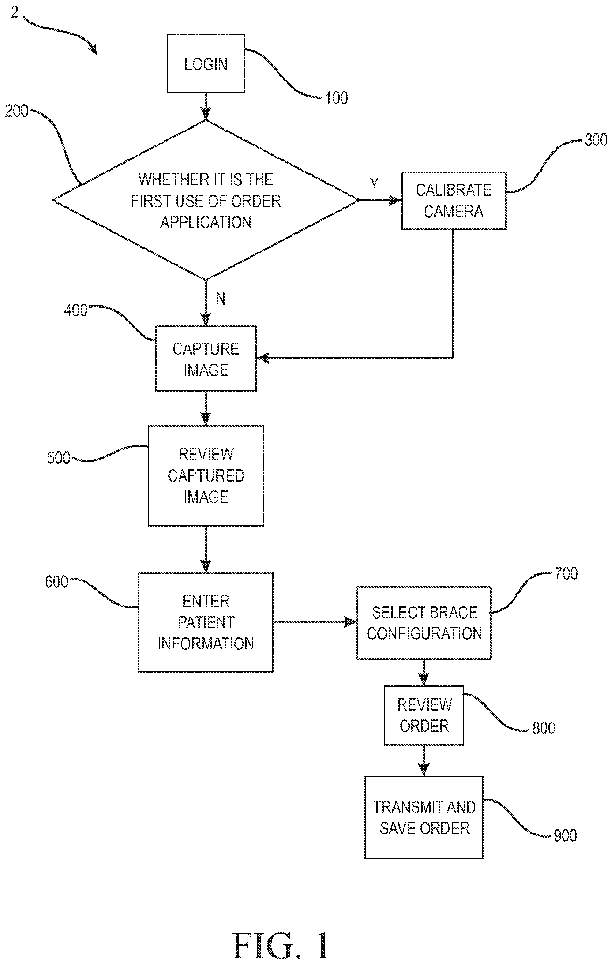

Fig. 1 is an overview of an embodiment of an embodiment of the method 2 for ordering a custom orthopedic device. After the user selects the ordering application, the application is launched, and the application displays a login screen. At step 100, the user logs into an account or creates a new account using a keypad or similar on the device. The login page may include an option for an embedded tutorial including a visual step-by-step discussion stored on the device or available to the device through streaming. The tutorial may provide guidance on patient positioning, making anatomical landmarks on the limb, and taking circumferential measurements.

At step 200, the application determines whether the current session is the first use of the device 4 to order the custom orthopedic device. In one embodiment, if the current session is the first use, the application calibrates a camera of the device at 300. During calibration 300, the application automatically sets the camera to specific settings such as a specific resolution, zoom, and color setting.

Alternatively, the calibration 300 of the camera may be omitted. In this embodiment, the preferred camera settings are in text within the application, and the calibration is manually performed by the user by adjusting the settings of the camera. The preferred camera settings may be displayed during the image capturing process and during a review of the captured image. The preferred camera settings can also be displayed and included in a "help" section of the application.

If the current session is not the first use or calibration of the camera is unnecessary, the application enables capture of an image using guidelines and a specific alignment or orientation at step 400. In step 500, the captured image is reviewed by the user to determine whether the captured image meets the specified alignment. At step 600, patient information is entered, and a brace configuration is selected at 700. The user is prompted to review the order at 800 before the order is saved on the device and transmitted over a network to a server of the custom orthopedic device manufacturer or seller at 900.

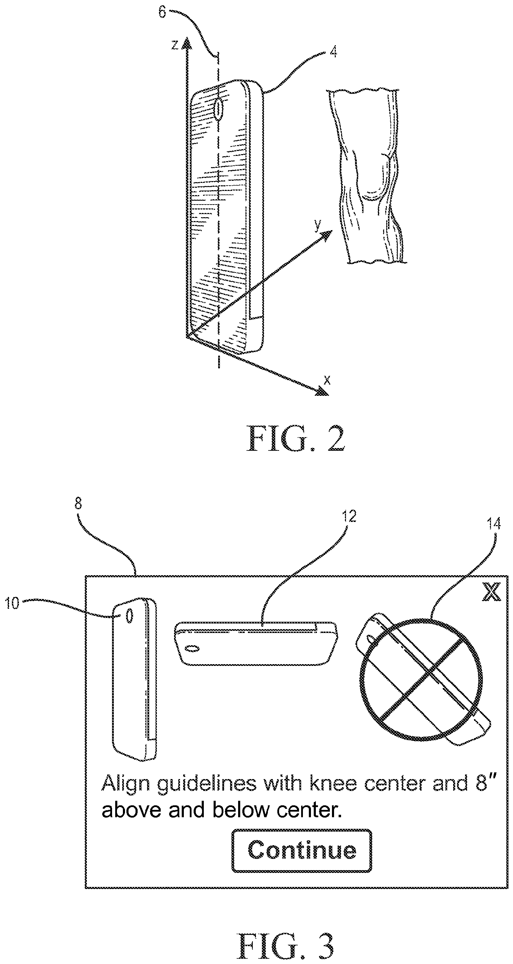

The capturing of the image of the limb at step 400 is described in more detail regarding Figs. 2-4. Before the application enables capturing of an image, the application ensures the device 4 is properly aligned with the limb. To produce a properly aligned image of the limb, the joint is preferably centered in the image, and the image is captured from a point at the same height as the joint with the plane of the image being parallel to the limb or a vertical axis while the device 4 is in a portrait or landscape orientation. Where the horizontal axis is parallel to the x-axis, the y-axis represents depth, and the vertical axis is parallel to the z-axis, a longitudinal axis 6 of the device would be parallel to the horizontal axis when in a landscape orientation or to the vertical axis when in a portrait orientation.

In determining whether the device 4 is in an acceptable portrait or landscape orientation, the application uses the gyroscope and/or accelerometer of the device 4 to determine whether the orientation of the device 4 is within a certain degree range, for example, whether the longitudinal axis 6 of the device 4 is within five degrees of the horizontal axis or within five degrees of the vertical axis.

The application provides a visual indication 8 on a display 18 of the device 4 whether the device 4 is properly oriented and allows the user to adjust the device 4 until the orientation requirements are met.

Fig. 3 shows the device in a portrait orientation 10 followed by a landscape orientation 12 and an unacceptable tilted orientation 14. When the device is within the certain degrees range of the landscape or portrait orientation, the display will indicate by highlighting the landscape or portrait orientation in Fig. 3. If the device is not within the certain degree range of the vertical axis or the device is otherwise rotated about the vertical or horizontal axes, the cross-out symbol will flash to indicate to the user that adjustment of the device is tilted and adjustment is required.

Once the orientation requirements are met, the display shows the viewfinder image and enables capture of an image. The display 18 of the device 4 becomes the viewfinder for the camera and a depth of field guideline 16 is overlaid on the viewfinder image. The depth of field guideline 16 provides the user with assistance in centering the joint in the photo and capturing the appropriate portion of the joint. Before capturing the image of the limb, the limb may be measured and marked at multiple points to indicate specific distances above and below the joint, to aid in alignment with the depth of field guideline, and to provide reference points for circumference measurements of the limb. The markings may be at specific points or at regular intervals along and around the limb.

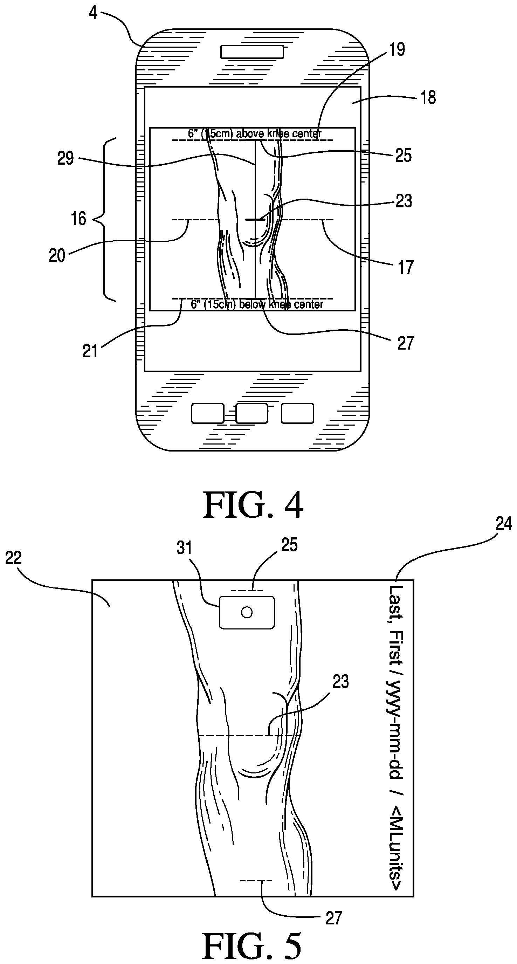

Fig. 4 shows an example of the depth of field guideline 16 on the display 18 of the device 4 to assist the user in capturing the appropriate portion of the limb. In the example, the portions 25, 27 of the leg about 15 cm above and below a knee axis 23 should be captured in the image for a custom knee brace. A hash mark 17 is provided at the center of the viewfinder image 20 to assist the user in aligning the joint with the center of the image. An upper guideline 19 is at the top of the viewfinder image 20 indicating that the distance of about 15 cm above the joint is aligned with the upper guideline 19. Similarly, a lower guideline 21 indicates the distance of about 15 cm below the joint is aligned with the lower guideline 21 in the image. The guideline 16, inclusive of mark and guidelines 17, 19, 21 include a vertical line 29 on the center of the image to align with the center of the limb. While the depth of field guideline 16 for the knee defines a portion of the knee about 15 cm above and below the knee, the depth of field guideline 16 defines a portion of the limb any distance above and below a certain point.

To aid the user in correctly framing the limb in the picture, markings are first placed on the limb indicating specific locations on the limb. An anatomical landmark or marking is placed at the point on the limb about 15 cm below the joint and about 15 cm above the joint which corresponds to the depth of field guidelines. Therefore, the photographer need only align the markings with the upper and lower guidelines and align the center hash mark over the center of the joint.

The method requires a delay before the image is taken to assure stable and clear focus. Upon alignment with the field guideline, the method requires a steady position before taking the image. A signal may be released, such as a green dot, to prompt the user to capture the image by pressing a button on the device.

In a variation, the clinician first identifies the medial joint space and marks it appropriately. The clinician then measures approximately 2 cm above the medial joint space and draws a line across the knee, from medial to lateral sides, to define the knee axis line. The clinician then identifies and marks the lateral joint space. The clinician then may measure and mark points both 7.5 cm and 15 cm above and below the knee axis line. The clinician marks the tibial peak below the 15 cm mark and the tibial peak above the 7.5 cm mark, and connects the two with a line.

The application may provide on-screen guides as to the current orientation of the device relative to the preferred orientation regarding each of the axes next to or over the viewfinder image.

In this manner, the application assists and guides the user in capturing the optimal image of the limb for a custom orthopedic device. Through immediate on-screen guidance as to the orientation of the device 4, the user can easily and quickly adjust the angle, orientation, and alignment of the device to obtain a well aligned and consistent photograph of the limb. The possibility of misalignment between the image capturing device and the limb is therefore greatly reduced.

According to a variation, the clinician takes both an anterior view of the limb with the markings, and a lateral view of the limb. In both instances, the depth of field guideline may vary according to the orientation of the limb, which is selected on the device by the clinician. The method may include image capture from any number sides of a patient's limb, including anterior, posterior, lateral and medial views and angles.

Once an image is captured, the user is prompted to review the captured image. During the review of the captured photograph at step 500, the captured image with the overlaid guidelines 16 is shown, and the user determines whether the correct portion of the limb is captured within the image and whether the limb is centered within the image. If the limb and the device 4 were not aligned when the image was captured, a new image may be taken. If a new image is captured, the application returns to step 400 to guide the user through the correct orientation and framing of the limb in the image. Once the photograph is confirmed, the user is prompted to enter measurements of the limb. The measurements of the limb may include the medial-lateral (M-L) width measurement of the limb and the circumference of the limb at various points above and below the joint. A measuring device such as a caliper or measuring tape may obtain the measurements of the limb.

To associate the captured image 22 with the patient, the captured image 22 is stored with an identification label 24. The identification label 24 may be text overlaid on the captured image 22. Fig. 5 is an example of a saved image overlaid with an identification label 24. The information label 24 may include data such as the patient's name, date of the photograph, and the M-L measurement along one side of the image. The image with overlaid information is stored in the memory of the device. The image may be password protected or encrypted in the memory to protect the patient's privacy.

As shown in Fig. 5, the system may include a tilt indicator 31 arranged to allow the clinician to correctly orient the camera position. The tilt indicator 31 relies on a determination by the device to measure whether the angle or orientation of the device is proper to assure a successful captured image. In a preferred embodiment, the tilt indicator will display a red color when the device is not in the proper orientation to capture the image, and a green color when the device is in a proper orientation.

At 600, additional patient information is entered to fill out the order form. The patient information may include the name of the user, the prescriber of the orthopedic device, and the diagnosis or symptoms of the user with identification of the problem joint. After the patient information is entered, the application can create a partial order on the device in encrypted XML format including the captured photo and patient information. Other security measures may protect the patient's data under HIPAA regulations.

At step 700, the user or patient selects the appropriate orthopedic device configuration such as the orthopedic device model and color. The application guides the user through the different orthopedic devices and provides the user with options based on the selected orthopedic device. After selection and configuration of the orthopedic device, the partial order is saved. The user can enter a menu listing all orders saved on the device or continue to a review of the order. The list also indicates the status of the order such as whether the order has been transmitted to the maker or seller. If the user selects a link for an order not yet transmitted, the user is prompted to review the order.

At step 800, the order is displayed for review. The saved image and the collected information are displayed. The user can edit portions of the order with the changes made during the review being saved with the order. The user can also enter the clinician information and payment and shipping information. The user then may save the order or transmitting the order.

At step 900, the order is saved and/or transmitted to the server of the custom orthopedic device provider. The application sends the order in an e-mail and automatically populates the fields of the e-mail based on the data in the saved order. The patient information, brace information, and clinician or user information are inserted into the body of the e-mail while the saved image associated with the order is automatically attached to or inserted into the e-mail. When the user elects to send the e-mail containing the order information, the device can send the order directly to a server of the seller through a network. The application and/or the server may display or send a notification to the user to confirm the order.

While the embodiments described relate to a method and device for ordering a custom orthopedic device which can be accomplished with a single image, in another embodiment the method and device are used with or within a custom orthopedic device production method and system which produces the custom orthopedic device based on a three-dimensional model of the limb generated from a plurality of captured images.

To generate a three-dimensional model, markers or reference points are placed on the limb and are subsequently captured in the image. The markers or reference points assist in determining the dimensions of the limb from the image by providing information related to the surface of the limb. To place the markers or reference points on the limb, a sock or sleeve having markings may be worn on the limb or the limb may be marked at particular intervals. Markings can be contrasting colored markings in many shapes such as a circular shape, a rectangular shape, a triangular shape, or any combination and are preferably the same size. The distribution and density of the markings over the surface of the sock, sleeve, or limb varies depending on the type of limb and the desired three-dimensional modeling resolution.

Providing more markings or a higher density of markings in a certain area produces a more accurate three-dimensional model of the limb since more reference points would be provided in the captured image. The markings may be concentrated in areas where there are more variations in the continuity of the limb surface such as around the joint area. Additional references may be added to the limb or the sock. In ordering a custom knee brace, additional markings or references are added to indicate the center of the knee, the angle of the tibia, and the locations of the condyles.

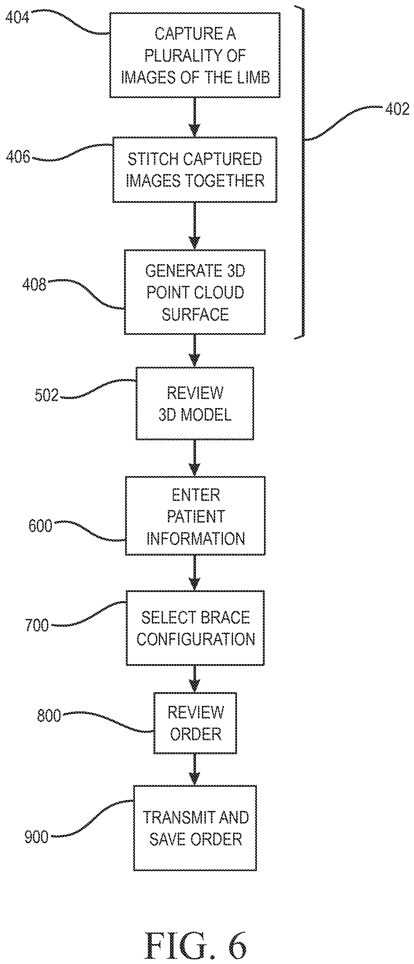

Fig. 6 shows an image capturing process 402 and a review stage 502 in an embodiment of the method for ordering a custom orthopedic device where a three-dimensional image of the limb is captured. This embodiment follows the same steps as the embodiment in Fig. 1 and differs in the specific steps taken to capture the image at 400 and in the image displayed during the review stage 500 of the embodiment of Fig. 1. After the three-dimensional model specific steps of 402 and 502, the ordering process continues with the remaining general ordering steps 600, 700, 800, and 900 as described with respect to Fig. 1.

Once the ordering application is executed and before the image capturing step 400, the user is prompted to select whether to use a three-dimensional model. If the user does not select the three-dimensional model, the method continues as described regarding Figs. 1-5. If the user uses the three-dimensional option, the image capturing process 402 as shown in Fig. 6 and review process 502 are used.

Before beginning the image capturing process 402, markings or reference points are added to the limb in the manner described. In the image capturing process 402 starting with step 404, the photographer captures at least two images of the limb at various angles or views. Preferably, at least four individual images are captured of the limb with each image capturing a different angle or view of the limb such that the entire circumference of the appropriate portion of the limb is captured within the plurality of images. The application may instruct the user to capture a certain number of images of the limb from different angles, orientations, heights, or different portions of the limb.

Alternatively, the application may use continuous image capturing where the application automatically captures images at different intervals such that the photographer need only move the device and indicate when images of all views or sides of the limb have been captured.

During the image capturing process 402, the application can guide the photographer in capturing the appropriate angles or views of the limb with the appropriate alignment of the limb in the image using accelerometer and/or gyroscope data from the device using the guidelines described. Depending on the depth of field of the images, the application can determine the appropriate number of images, angles, or views needed to generate an accurate three-dimensional model of the limb.

At step 406, the application analyzes and processes the plurality of captured images to stitch the images together and form a continuous view of the limb. The application can perform the stitching of the images together automatically or with user assistance. The stitching of the images can also be performed while the images are being captured or with the plurality of individual separately captured images.

At 408, from the stitched image, the application generates a three-dimensional model of the limb using the markings on the sock, sleeve, or limb. The model may be a computer-aided design (CAD) point cloud surface where the markings shown in the captured images translate into points in the point could surface. The application preferably generates a 360° view of the limb.

Once the image capturing process 402 is completed, the application prompts the user to review the generated three-dimensional model to ensure that the three-dimensional model accurately depicts the surface shape of the corresponding portion of the limb at 502. During review of the three-dimensional model, the user can rotate and zoom into the model to view all sides of and different levels of detail of the limb. The user is given the option of approving the generated model or re-capturing the limb to generate a more accurate model.

If the user performs the image capturing process 402 again, the current generated model may be saved for comparison with later models during the review stage. If the user approves of the generated model, the application continues with the general ordering process stages 600-900 as described regarding Fig. 1. For the order review at 800 and transmission and saving of the order 900, the order can include the plurality of images and/or the three-dimensional model of the limb.

Many of the elements described in the disclosed embodiments may be implemented as modules. A module is defined here as an isolatable element that performs a defined function and has a defined interface to other elements. The modules described in this disclosure may be implemented in hardware, a combination of hardware and software, firmware, or a combination, all of which are behaviorally equivalent.

Modules may be implemented using computer hardware in combination with software routine(s) written in a computer language (such as C, C++, Fortran, Java, Basic, Matlab or the like). It may be possible to implement modules using physical hardware that incorporates discrete or programmable analog and/or digital hardware. Examples of programmable hardware include: computers, microcontrollers, microprocessors, application-specific integrated circuits (ASICs); field programmable gate arrays (FPGAs); and complex programmable logic devices (CPLDs). Computers, microcontrollers and microprocessors are programmed using languages such as assembly, C, C++ or the like. Finally, the above mentioned technologies may be used in combination to achieve the result of a functional module.

The application may be software embodied on a computer readable medium which when executed by a processor of a computer performs a sequence of steps. A computer readable medium may be a floppy disk, a flexible disk, a hard disk, magnetic tape, or any other magnetic medium, a CD-ROM, any other optical medium, a RAM, a ROM, or any other medium from which a computer can read. Various forms of computer readable media may carry one or more sequences of one or more instructions to a processor for execution. The software may be transmitted over a wired or wireless network to the device.

A preferred order for the steps in the method of ordering the custom orthopedic device has been described. It is noted that the order of the steps in the method may be rearranged.

## Claims

1. A method (2) reducing image and limb misalignment in capturing an image of the limb for ordering a custom orthopedic device for a joint of the limb, comprising:
aligning a viewfinder image (20) displayed on a display (18) and generated by an image sensor of a portable device (4) with at least one predetermined portion of the limb including the joint;
capturing and storing an image of the portion of the limb using the image sensor of the portable device (4) based on at least one limb alignment guideline (16, 19, 21, 29);
associating the captured image with measurements of the limb (23, 25, 27), and patient information entered into the portable device (4); and
transmitting an order containing the captured image, the measurements of the limb (23, 25, 27), and the patient information from the portable device (4) to a server of the custom orthopedic device provider; **wherein,**
the at least one limb alignment guideline (16, 19, 21, 29) is a depth of field guideline (16) overlaid on the viewfinder image (20), the depth of field guideline (16) defining an upper guideline (19) for a first predetermined distance above the joint, a lower guideline (21) for a second predetermined distance below the joint, a hash mark (17) at a center of the viewfinder image (20) for a joint axis (23), and a vertical line (29) arranged on the center of the viewfinder image (20) for a center of the limb;
before capturing of the image is enabled, it is determined that orientation of the device (4) requirements are met to ensure that the device (4) is properly aligned with the limb for producing a properly aligned image of the limb, in which the j oint is centered in the image and the image is captured from a point at the same height as the joint with the plane of the image being parallel to the limb or the vertical axis while the device (4) is in a portrait or landscape orientation, wherein the horizontal axis defines an x-axis, a y-axis represents depth, and the vertical axis defines a z-axis, and wherein the longitudinal axis (6) of the device (4) is parallel to the horizontal axis when in landscape orientation (12) or to the vertical axis when in portrait orientation (10);
in determining whether the device (4) is in an acceptable portrait orientation (10) or landscape orientation (12):
- a gyroscope and/or an accelerometer of the device (4) is used to determine whether the orientation of the device (4) is within a certain degree range of the horizontal axis or of the vertical axis;
- a visual indication (8) is provided on the display (18) of the device (4) indicating whether the device (4) is properly oriented thereby allowing adjusting of the device (4) until the orientation requirements are met, wherein, when the device (4) is within the certain degree range of the landscape orientation (12) or the portrait orientation (10), the orientation is indicated on the display (18) otherwise it is indicated that the adjustment of the device (4) is tilted and adjustment is required; and
- once the orientation requirements are met, the viewfinder image (20) is shown on the display (18) and capturing of the image is enabled.

2. The method according to claim 1, wherein the first and second predetermined distances are the same and referenced from the joint axis (23).

3. The method according to claim 1, further comprising the step of:
aligning the first and second predetermined distances above and below the joint axis (23) with the depth of field guideline (16) shown in the viewfinder image (20) before capturing the image.

4. The method according to claim 1, wherein once the orientation of the portable device (4) relative to the limb satisfies the at least one limb alignment guideline (16, 19, 21, 29), the portable device (4) enables image capture.

5. The method according to any one of the preceding claims, further comprising the step of calibrating the image sensor of the portable device (4).

6. The method according to any one of the preceding claims, further comprising the steps of:
executing an ordering application;
determining whether the ordering application has been previously executed;
upon the determination that the ordering application has not been previously executed, calibrating the image sensor of the portable device (4); and
upon the determination that the ordering application has been previously executed, enabling capture of the image of the limb.

7. The method according to any one of the preceding claims, wherein at least two images are captured at two different angles or sides of the limb.

8. The method according to claim 7, wherein the step of reviewing the captured image of the limb, comprises the steps of:
viewing the captured image with an overlaid depth of field guideline (16) to confirm the captured portion of the limb satisfies the overlaid depth of field guideline (16);
entering basic patient information into the portable device (4), wherein the basic patient information includes measurements of the limb at various locations on the limb;
overlaying the captured image with the basic patient information; and
storing the overlaid captured image in the portable device (4).

9. The method according to any one of the preceding claims, further comprising the steps of:
configuring the custom orthopedic device;
reviewing the order; and
storing the order in a memory of the portable device (4),wherein preferably at least one previous order is stored in the memory of the portable device (4).

10. The method according to any one of the preceding claims, wherein the order is transmitted as an e-mail containing the patient information and the saved, captured image of the limb.

11. The method according to any one of the preceding claims, wherein the joint is a knee joint and the orthopedic device is a knee brace.

12. A device (4) for ordering a custom orthopedic device, comprising: an image sensor, the image sensor configured to capture an image; a display (18); a communication interface; a processor; and a memory;
wherein the processor is configured to enable capturing an image of a portion of a limb including a joint using the image sensor based on at least one limb alignment guideline (16, 19, 21, 29) in a viewfinder image (20) displayed on the display (18), if the image of the limb satisfies the at least one limb alignment guideline;
wherein the communication interface is configured to transmit an order containing the captured image and patient information from the apparatus over a network to a provider; wherein
the device (4) further comprises a gyroscope and/or accelerometer configured to provide orientation data to the processor; and
the at least one limb alignment guideline (16, 19, 21, 29) is a depth of field guideline (16) overlaid on the viewfinder image (20), the depth of field guideline (16) defining an upper guideline (19) for a first predetermined distance above the joint, a lower guideline (21)_for a second predetermined distance below the joint, a hash mark (17) at a center of the viewfinder image (20) for a joint axis (23), and a vertical line (29) arranged on the center of the viewfinder image (20) for a center of the limb; and
the processor is further configured
before enabling the capturing of the image of the portion of the limb, to assist an user of the device (4) in aligning the device (4) with the limb by
- determining that orientation of the device (4) requirements are met to ensure that the device (4) is properly aligned with the limb for producing a properly aligned image of the limb, in which the joint is centered in the image and the image is captured from a point at the same height as the joint with the plane of the image being parallel to the limb or the vertical axis while the device (4) is in a portrait or landscape orientation, wherein the horizontal axis defines an x-axis, a y-axis represents depth, and the vertical axis defines a z-axis, and wherein the longitudinal axis (6) of the device (4) is parallel to the horizontal axis when in landscape orientation (12) or to the vertical axis when in portrait orientation (10);
wherein in determining whether the device (4) is in an acceptable portrait orientation (10) or landscape orientation (12):
- to use the orientation data provided by the gyroscope and/or an accelerometer to determine whether the orientation of the device (4) is within a certain degree range of the horizontal axis or of the vertical axis;
- to provide a visual indication (8) on the display (18) of the device (4) indicating whether the device (4) is properly oriented thereby allowing adjusting of the device (4) until the orientation requirements are met, wherein, when the device (4) is within the certain degree range of the landscape orientation (12) or the portrait orientation (10), to indicate the orientation on the display (18) otherwise to indicate that the adjustment of the device (4) is tilted and adjustment is required; and
- once the orientation requirements are met, to show the viewfinder image (20) on the display (18) and to enable the capturing of the image.

13. Computer program product in the form of an ordering application software comprising instructions to cause the device according to claim 12 to execute the steps of the method of any of claims 1 to 11.

## Patentansprüche

1. Verfahren (2) zur Reduzierung der Fehlausrichtung eines Bilds und eines Gliedmaßes bei der Aufnahme eines Gliedmaß-Bildes für die Bestellung einer kundenspezifischen orthopädischen Vorrichtung für ein Gliedmaß-Gelenk, mit:
Ausrichten eines Sucherbildes (20), das auf einer Anzeige (18) angezeigt und von einem Bildsensor einer tragbaren Vorrichtung (4) erzeugt wird, mit mindestens einem vorbestimmten Teil des Gliedmaßes, welches das Gelenk aufweist;
Erfassen und Speichern eines Bildes des Teils des Gliedmaßes unter Verwendung des Bildsensors der tragbaren Vorrichtung (4) auf der Grundlage von mindestens einer Gliedmaß-Ausricht-Leitlinie (16, 19, 21, 29);
Verknüpfen des aufgenommenen Bildes mit Messungen des Gliedmaßes (23, 25, 27) und in die tragbare Vorrichtung (4) eingegebenen Patienteninformationen; und
Übertragen eines Auftrags, der das aufgenommene Bild, die Maße des Gliedmaßes (23, 25, 27) und die Patienteninformationen enthält, von der tragbaren Vorrichtung (4) an einen Server des Anbieters der individuellen orthopädischen Vorrichtung; wobei,
die mindestens eine Gliedmaß-Ausricht-Leitlinie (16, 19, 21, 29) eine Schärfentiefe-Leitlinie (16) ist, die dem Sucherbild (20) überlagert ist, wobei die Schärfentiefe-Leitlinie (16) eine obere Leitlinie (19) für einen ersten vorbestimmten Abstand oberhalb des Gelenks definiert, eine untere Leitlinie (21) für einen zweiten vorbestimmten Abstand unterhalb des Gelenks, eine Raute (17) in der Mitte des Sucherbildes (20) für eine Gelenkachse (23) und eine vertikale Linie (29), die in der Mitte des Sucherbildes (20) für ein Zentrum des Gliedmaßes angeordnet ist;
bevor die Aufnahme des Bildes freigegeben wird, bestimmt wird, dass die Anforderungen an die Ausrichtung der Vorrichtung (4) erfüllt sind, um sicherzustellen, dass die Vorrichtung (4) richtig auf das Gliedmaß ausgerichtet ist, um ein richtig ausgerichtetes Bild des Gliedmaßes zu erzeugen, in dem das Gelenk in dem Bild zentriert ist und das Bild von einem Punkt auf der gleichen Höhe wie das Gelenk aufgenommen wird, wobei die Ebene des Bildes parallel zu dem Gliedmaß oder der vertikalen Achse ist, während die Vorrichtung (4) sich in einer Hoch- oder Querformatausrichtung befindet, wobei die horizontale Achse eine x-Achse definiert, eine y-Achse die Tiefe darstellt und die vertikale Achse eine z-Achse definiert, und wobei die Längsachse (6) der Vorrichtung (4) parallel zur Horizontalachse ist, wenn sie sich im Querformat (12) befindet, oder zur vertikalen Achse, wenn sie sich im Hochformat (10) befindet;
beim Bestimmen, ob sich die Vorrichtung (4) in einer akzeptablen Hochformatausrichtung (10) oder Querformatausrichtung (12) befindet:
- ein Gyroskop und/oder ein Beschleunigungsmesser des Geräts (4) verwendet wird, um zu bestimmen, ob die Ausrichtung des Geräts (4) innerhalb eines bestimmten Gradbereichs der horizontalen Achse oder der vertikalen Achse liegt;
- eine visuelle Anzeige (8) auf der Anzeige (18) der Vorrichtung (4) bereitgestellt wird, die angibt, ob die Vorrichtung (4) richtig ausgerichtet ist, wodurch ein Einstellen der Vorrichtung (4) ermöglicht wird, bis die Ausrichtungsanforderungen erfüllt sind, wobei die Ausrichtung auf der Anzeige (18) angezeigt wird, wenn sich die Vorrichtung (4) innerhalb des bestimmten Gradbereichs der Querformatausrichtung (12) oder der Hochformatausrichtung (10) befindet, andernfalls wird angezeigt, dass die Einstellung der Vorrichtung (4) geneigt ist und ein Einstellen erforderlich ist; und
- sobald die Anforderungen an die Ausrichtung erfüllt sind, das Sucherbild (20) auf dem Display (18) angezeigt und das Aufnehmen des Bildes wird aktiviert wird.

2. Verfahren nach Anspruch 1, wobei der erste und der zweite vorbestimmte Abstand gleich sind und sich auf die Gelenkachse (23) beziehen.

3. Verfahren nach Anspruch 1, ferner mit dem Schritt:
Ausrichten der ersten und zweiten vorbestimmten Abstände oberhalb und unterhalb der Gelenkachse (23) mit der Schärfentiefe-Leitlinie (16), die im Sucherbild (20) angezeigt wird, vor dem Aufnehmen des Bildes.

4. Verfahren nach Anspruch 1, wobei das tragbare Gerät (4) die Bildaufnahme ermöglicht, sobald die Ausrichtung des tragbaren Geräts (4) relativ zum Gliedmaß die mindestens eine Gliedmaß-Ausricht-Leitlinie (16, 19, 21, 29) erfüllt.

5. Verfahren nach einem der vorhergehenden Ansprüche ferner mit dem Schritt des Kalibrierens des Bildsensors des tragbaren Geräts (4).

6. Verfahren nach einem der vorhergehenden Ansprüche, ferner mit den Schritten:
Ausführen einer Bestellanwendung;
Bestimmen, ob die Bestellanwendung zuvor ausgeführt worden ist;
bei der Bestimmung, dass die Bestellanwendung nicht vorher ausgeführt wurde, Kalibrieren des Bildsensors der tragbaren Vorrichtung (4); und
nach der Bestimmung, dass die Bestellanwendung zuvor ausgeführt wurde, Ermöglichen der Aufnahme des Bildes des Gliedmaßes.

7. Verfahren nach einem der vorhergehenden Ansprüche, wobei mindestens zwei Bilder aus zwei verschiedenen Winkeln oder von zwei verschiedenen Seiten des Gliedmaßes aufgenommen werden.

8. Verfahren nach Anspruch 7, wobei der Schritt des Überprüfens des aufgenommenen Bildes des Gliedmaßes die folgenden Schritte aufweist:
Betrachten des aufgenommenen Bildes mit einer überlagerten Schärfentiefe-Leitlinie (16), um zu bestätigen, dass das aufgenommene Teil des Gliedes der überlagerten Schärfentiefe-Leitlinie (16) entspricht;
Eingeben grundlegender Patienteninformationen in die tragbare Vorrichtung (4), wobei die grundlegenden Patienteninformationen Messungen des Gliedmaßes an verschiedenen Stellen des Gliedmaßes aufweisen;
Überlagern des aufgenommenen Bildes mit den grundlegenden Patienteninformationen; und
Speichern des überlagerten aufgenommenen Bildes in der tragbaren Vorrichtung (4).

9. Verfahren nach einem der vorhergehenden Ansprüche, ferner mit den Schritten:
Konfigurieren der individuellen orthopädischen Vorrichtung;
Überprüfen der Bestellung; und
Speichern der Bestellung in einem Speicher der tragbaren Vorrichtung (4),wobei vorzugsweise mindestens eine vorherige Bestellung in dem Speicher der tragbaren Vorrichtung (4) gespeichert ist.

10. Verfahren nach einem der vorhergehenden Ansprüche, wobei die Bestellung als E-Mail mit den Patienteninformationen und dem gespeicherten, aufgenommenen Bild des Gliedmaßes übermittelt wird.

11. Verfahren nach einem der vorhergehenden Ansprüche, wobei das Gelenk ein Kniegelenk und die orthopädische Vorrichtung eine Kniestütze ist.

12. Vorrichtung (4) zum Bestellen einer kundenspezifischen orthopädischen Vorrichtung, mit: einem Bildsensor, wobei der Bildsensor konfiguriert ist, ein Bild aufzunehmen; einer Anzeige (18); einer Kommunikationsschnittstelle; einem Prozessor; und einem Speicher;
wobei der Prozessor konfiguriert ist, das Aufnehmen eines Bildes eines Teils eines Gliedma-βes einschließlich eines Gelenks unter Verwendung des Bildsensors basierend auf mindestens einer Gliedmaß-Ausricht-Leitlinie (16, 19, 21, 29) in einem auf dem Display (18) angezeigten Sucherbild (20) zu ermöglichen, wenn das Bild des Gliedmaßes die mindestens eine Gliedmaß-Ausricht-Leitlinie erfüllt;
wobei die Kommunikationsschnittstelle konfiguriert ist, einen Auftrag, der das aufgenommene Bild und Patienteninformationen enthält, von der Vorrichtung über ein Netzwerk an einen Anbieter zu übertragen; wobei
die Vorrichtung (4) ferner ein Gyroskop und/oder einen Beschleunigungsmesser aufweist, die konfiguriert sind, Orientierungsdaten für den Prozessor bereitzustellen; und
die mindestens eine Gliedmaß-Ausricht-Leitlinie (16, 19, 21, 29) eine Schärfentiefe-Leitlinie (16) ist, die dem Sucherbild (20) überlagert ist, wobei die Schärfentiefe-Leitlinie (16) eine obere Leitlinie (19) für einen ersten vorbestimmten Abstand oberhalb des Gelenks definiert, eine untere Leitlinie (21) für einen zweiten vorbestimmten Abstand unterhalb des Gelenks, eine Raute (17) in der Mitte des Sucherbildes (20) für eine Gelenkachse (23) und eine vertikale Linie (29), die in der Mitte des Sucherbildes (20) für ein Zentrum des Gliedmaßes angeordnet ist; und
der Prozessor ferner konfiguriert ist,
vor dem Ermöglichen des Erfassens des Bildes des Teils des Gliedmaßes einen Benutzer der Vorrichtung (4) beim Ausrichten der Vorrichtung (4) mit dem Gliedmaß zu unterstützen, durch
- Bestimmen, dass die Anforderungen an die Ausrichtung der Vorrichtung (4) erfüllt sind, um sicherzustellen, dass die Vorrichtung (4) richtig auf das Gliedmaß ausgerichtet ist, um ein richtig ausgerichtetes Bild des Gliedmaßes zu erzeugen, in dem das Gelenk im Bild zentriert ist und das Bild von einem Punkt auf der gleichen Höhe wie das Gelenk aufgenommen wird, wobei die Ebene des Bildes parallel zum Gliedmaß oder zur vertikalen Achse verläuft, während die Vorrichtung (4) im Hoch- oder Querformat ausgerichtet ist, wobei die horizontale Achse eine x-Achse definiert, eine y-Achse die Tiefe darstellt und die vertikale Achse eine z-Achse definiert, und wobei die Längsachse (6) der Vorrichtung (4) parallel zur horizontalen Achse verläuft, wenn sie im Querformat (12) ist, oder zur vertikalen Achse, wenn sie im Hochformat (10) ist;
wobei beim Bestimmen, ob die Vorrichtung (4) in einer akzeptablen Hochformatausrichtung (10) oder Querformatausrichtung (12) ist,
- die von dem Gyroskop und/oder einem Beschleunigungsmesser bereitgestellten Ausrichtungsdaten zu verwenden, um zu bestimmen, ob die Ausrichtung des Geräts (4) innerhalb eines bestimmten Gradbereichs der horizontalen Achse oder der vertikalen Achse liegt;
- eine visuelle Anzeige (8) auf der Anzeige (18) der Vorrichtung (4) bereitzustellen, die angibt, ob die Vorrichtung (4) richtig ausgerichtet ist, wodurch ein Einstellen der Vorrichtung (4) ermöglicht wird, bis die Ausrichtungsanforderungen erfüllt sind, wobei, wenn die Vorrichtung (4) innerhalb des bestimmten Gradbereichs der Querformatausrichtung (12) oder der Hochformatausrichtung (10) liegt, die Ausrichtung auf der Anzeige (18) angezeigt wird, um andernfalls anzuzeigen, dass die Einstellung der Vorrichtung (4) gekippt ist und ein Einstellen erforderlich ist; und
- sobald die Anforderungen an die Ausrichtung erfüllt sind, das Sucherbild (20) auf dem Display (18) anzuzeigen und die Aufnahme des Bildes zu ermöglichen.

13. Computerprogrammprodukt in der Form einer Bestellanwendungssoftware, die Anweisungen aufweist, um die Vorrichtung nach Anspruch 12 zu veranlassen, die Schritte des Verfahrens eines der Ansprüche 1 bis 11 auszuführen.

## Revendications

1. Procédé (2) destiné à réduire un désalignement d'image et de membre lors de la capture d'une image du membre pour commander un dispositif orthopédique sur mesure pour une articulation du membre, comprenant les étapes suivantes :
aligner une image de viseur (20) affichées sur un affichage (18) et générée par un capteur d'image d'un dispositif portable (4), au moins une portion prédéterminée du membre incluant l'articulation ;
capturer et stocker une image de la portion du membre à l'aide du capteur d'image du dispositif portable (4) sur la base d'au moins une ligne de guidage pour alignement de membre (16, 19, 21, 29) ;
associer l'image capturée avec des mesures du membre (23, 25, 27) et des informations de patient saisies dans le dispositif portable (4), et
transmettre une commande contenant l'image capturée, les mesures du membre (23, 25, 27), et les informations de patient du dispositif portable (4) à un serveur du fournisseur de dispositif orthopédique sur mesure, dans lequel
la au moins une ligne de guidage pour alignement de membre (16, 19, 21, 29) est une profondeur de ligne de guidage de champ (16) superposée sur l'image de viseur (20), la profondeur de la ligne de guidage de champ (16) définissant une ligne de guidage supérieure (19) pour une première distance prédéterminée au-dessus de l'articulation, une ligne de guidage inférieure (21) pour une seconde distance prédéterminée au-dessous de l'articulation, une marque de graduation (17) en un centre de l'image de viseur (20) pour un axe d'articulation (23), et une ligne verticale (29) agencée sur le centre de l'image de viseur (20) pour un centre du membre ;
avant activation de la capture de l'image, il est déterminé que les exigences d'orientation du dispositif (4) sont satisfaites afin de s'assurer que le dispositif (4) est correctement aligné avec le membre pour produire une image correctement alignée du membre, où l'articulation est centrée dans l'image et l'image est capturée à partir d'un point sur la même hauteur que l'articulation, le plan de l'image étant parallèle au membre ou à l'axe vertical tandis que le dispositif (4) se trouve dans une orientation portrait ou paysage, dans lequel l'axe horizontal définit un axe x, un axe y définit une profondeur, et l'axe vertical définit un axe z, et dans lequel l'axe longitudinal (6) du dispositif (4) est parallèle à l'axe horizontal lorsque dans l'orientation paysage (12), ou à l'axe vertical lorsque dans l'orientation portrait (10) ;
lors de la détermination de si oui ou non le dispositif (4) se trouve dans une orientation portrait (10) ou une orientation paysage (12) acceptable :
- un gyroscope ou un accéléromètre du dispositif (4) est utilisé pour déterminer si oui ou non l'orientation du dispositif (4) se trouve dans les limites d'une certaine plage de degrés de l'axe horizontal ou de l'axe vertical ;
- une indication visuelle (8) est fournie sur l'affichage (18) du dispositif (4) indiquant si oui ou non le dispositif (4) est orienté correctement, ceci permettant un ajustement du dispositif (4) jusqu'à ce que les exigences d'orientation soient satisfaites, dans lequel lorsque le dispositif (4) se trouve dans les limites de la certaine plage de degrés de l'orientation paysage (12) ou de l'orientation portrait (10), l'orientation est indiquée sur l'affichage (18), sinon, il est indiqué que l'ajustement du dispositif (4) est incliné et qu'un ajustement est requis, et
- une fois que les exigences d'orientation sont satisfaites, l'image de viseur (20) est montrée sur l'affichage (18) et la capture de l'image est activée.

2. Procédé selon la revendication 1, dans lequel les première et seconde distances prédéterminées sont identiques et référencées à partir de l'axe d'articulation (23).

3. Procédé selon la revendication 1, comprenant en outre l'étape suivante :
aligner les première et seconde distances prédéterminées au-dessus et au-dessous de l'axe d'articulation (23), la profondeur de la ligne de guidage de champ (16) étant montrée dans l'image de viseur (20) avant capture de l'image.

4. Procédé selon la revendication 1, dans lequel une fois que l'orientation du dispositif portable (4) par rapport au membre satisfait la au moins une ligne de guidage pour alignement de membre (16, 19, 21, 29), le dispositif portable (4) active la capture d'image.

5. Procédé selon l'une quelconque des revendications précédentes, comprenant en outre l'étape pour étalonner le capteur d'image du dispositif portable (4).

6. Procédé selon l'une quelconque des revendications précédentes, comprenant en outre l'étape pour :
exécuter une application de commande ;
déterminer si oui ou non l'application de commande a été exécutée au préalable ;
lors de la détermination suivant laquelle l'application de commande n'a pas été exécutée au préalable, étalonner le capteur d'image du dispositif portable (4), et
lors de la détermination suivant laquelle l'application de commande a été exécutée au préalable, activer la capture de l'image du membre.

7. Procédé selon l'une quelconque des revendications précédentes, dans lequel au moins deux images sont capturées sur deux angles différents ou sur les côtés du membre.

8. Procédé selon la revendication 7, dans lequel l'étape pour examiner l'image capturée du membre comprend les étapes pour :
visualiser l'image capturée avec une profondeur de superposition de la ligne de guidage de champ (16) pour confirmer que la portion capturée du membre satisfait la profondeur de superposition de la ligne de guidage de champ (16) ;
saisir des informations de patient de base dans le dispositif portable (4), dans lequel les informations de patient de base incluent des mesures du membre en divers endroits sur le membre ;
superposer l'image capturée avec les informations de patient de base, et
stocker l'image capturée de superposition dans le dispositif portable (4).

9. Procédé selon l'une quelconque des revendications précédentes, comprenant en outre les étapes pour :
configurer le dispositif orthopédique sur mesure ;
examiner la commande, et
stocker la commande dans une mémoire du dispositif portable (4), dans lequel de préférence, au moins une commande précédente est stockée dans la mémoire du dispositif portable (4).

10. Procédé selon l'une quelconque des revendications précédentes, dans lequel la commande est transmise sous la forme d'un courriel contenant les informations de patient et l'image capturée et sauvegardée du membre.

11. Procédé selon l'une quelconque des revendications précédentes, dans lequel l'articulation est une articulation du genou, et le dispositif orthopédique est une orthèse de genou.

12. Dispositif (4) pour commander un dispositif orthopédique sur mesure, comprenant : un capteur d'image, le capteur d'image étant configuré pour capturer une image, un affichage (18), une interface de communication, un processeur, et une mémoire ;
dans lequel le processeur est configuré pour activer la capture d'une image d'une portion d'un membre incluant une articulation à l'aide du capteur d'image sur la base d'au moins une ligne de guidage pour alignement de membre (16, 19, 21, 29) dans une image de viseur (20) affichée sur l'affichage (18), si l'image du membre satisfait la au moins une ligne de guidage d'alignement de membre ;
dans lequel l'interface de communication est configurée pour transmettre une commande contenant l'image capturée et les informations de patient de l'appareil à un fournisseur via un réseau,
dans lequel
le dispositif (4) comprend en outre un gyroscope et/ou un accéléromètre configuré pour fournir des données d'orientation au processeur, et
la au moins une ligne de guidage pour alignement de membre (16, 19, 21, 29) est une profondeur de ligne de guidage de champ (16) superposée sur l'image de viseur (20), la profondeur de la ligne de guidage de champ (16) définissant une ligne de guidage supérieure (19) pour une première distance prédéterminée au-dessus de l'articulation, une ligne de guidage inférieure (21) pour une seconde distance prédéterminée au-dessous de l'articulation, une marque de graduation (17) en un centre de l'image de viseur (20) pour un axe d'articulation (23), et une ligne verticale (29) agencée sur le centre de l'image de viseur (20) pour un centre du membre, et
le processeur est en outre configuré pour
avant activation de la capture de l'image de la portion du membre, aider un utilisateur du dispositif (4) à aligner le dispositif (4) avec le membre
- déterminer si les exigences d'orientation du dispositif (4) sont satisfaites afin de s'assurer que le dispositif (4) est correctement aligné avec le membre pour produire une image correctement alignée du membre, où l'articulation est centrée dans l'image et l'image est capturée à partir d'un point sur la même hauteur que l'articulation, le plan de l'image étant parallèle au membre ou à l'axe vertical tandis que le dispositif (4) se trouve dans une orientation portrait ou paysage, dans lequel l'axe horizontal définit un axe x, un axe y définit une profondeur, et l'axe vertical définit un axe z, et dans lequel l'axe longitudinal (6) du dispositif (4) est parallèle à l'axe horizontal lorsque dans l'orientation paysage (12) ou à l'axe vertical lorsque dans l'orientation portrait (10) ;
dans lequel lors de la détermination de si oui ou non le dispositif (4) se trouve dans une orientation portrait (10) ou une orientation paysage (12) :
- utiliser les données d'orientation fournies par le gyroscope et/ou un accéléromètre pour déterminer si oui ou non l'orientation du dispositif (4) se trouve dans les limites d'une certaine plage de degrés de l'axe horizontal ou de l'axe vertical ;
- fournir une indication visuelle (8) sur l'affichage (18) du dispositif (4) indiquant si oui ou non le dispositif (4) est orienté correctement, ceci permettant un ajustement du dispositif (4) jusqu'à ce que les exigences d'orientation soient satisfaites, dans lequel lorsque le dispositif (4) se trouve dans les limites de la certaine plage de degrés de l'orientation paysage (12) ou de l'orientation portrait (10), pour indiquer l'orientation sur l'affichage (18), sinon pour indiquer que l'ajustement du dispositif (4) est incliné, un ajustement est requis, et
- une fois que les exigences d'orientation sont satisfaites, montrer l'image de viseur (20) sur l'affichage (18) et activer la capture de l'image.

13. Produit programme d'ordinateur, se présentant sous la forme d'un logiciel d'application de commande comprenant des instructions pour faire en sorte que le dispositif selon la revendication 12 exécute les étapes du procédé selon l'une quelconque des revendications 1 à 11.
